# EUROPEAN PATENT SPECIFICATION

(11) **EP 1 948 126 B1**
(45) Date of publication and mention of the grant of the patent: **26.12.2012**
(21) Application number: 06839512.8
(22) Date of filing: 24.10.2006
(51) Int. Cl.: A61K 8/49, A61Q 11/00

(54) **ORAL COMPOSITION CONTAINING MORIN**
MUNDPFLEGEZUSAMMENSETZUNG MIT MORIN
COMPOSITION ORALE CONTENANT DE LA MORINE

(30) Priority: 24.10.2005 US 256784
(43) Date of publication of application: 30.07.2008
(73) Proprietor: Colgate-Palmolive Company, New York NY 10022-7499 (US)
(72) Inventor: CAMERON, Ryan, B., Somerset, NJ 08873 (US); SUBRAMANYAM, Ravi, Belle Mead, NJ 08502 (US); TRIVEDI, Harsh, Sommerset, NJ 08873 (US); WORRELL, Cortney, North Plainfield, NJ 07060 (US)
(74) Representative: Jenkins, Peter David
(86) International application number: PCT/US2006/060175
(87) International publication number: WO 2007/051098

(56) References cited:
- EP-A2- 0 514 966
- DATABASE WPI Week 200162 Derwent Publications Ltd., London, GB; AN 2001-555269 XP002423731 & KR 2001 001 476 A (LG CHEM CO LTD) 5 January 2001 (2001-01-05)

## Description

This application relates to oral compositions.

### BACKGROUND OF THE INVENTION

Periodontal disease is inflammation of some or all of the tooth's support structures such as gingiva, cementum, periodontal ligament, and alveolar bone. The inflammation which generally results from infection of bacteria destroys the attachment fibers and supporting bone that hold the teeth in the mouth, leading to loss of teeth. Gingivitis and periodontitis are the most common periodontal diseases.

Among various factors causing periodontal diseases, oxidative cell damage is increasingly recognized as a source of tissue damage in the host and leads to inflammation. Oxidative free radicals are used by the body as defense systems against antigen attacks. However, when the response by the host is uncontrolled it leads to damage to tissues of the host such as seen in oral gingivitis. Therefore, an anti-oxidant that may suppress oxidative free radicals may provide a beneficial effect in mitigating inflammation processes of dental-related diseases.

EP-A-0514966 discloses dentifrice compositions containing stannous compounds which may also contain anti-oxidants such as morin, in particular dentifrices comprising an effective amount of a stannous compound capable of yielding stannous ions upon association with water, and an effective amount of a compound that is a radical inhibitor capable of reducing or preventing the conversion of the stannous ions in the dentifrice composition into stannic ions, wherein the antioxidant is morin. However, this publication does not disclose use of an antibacterial enhancing agent in a dentifrice to prevent or reduce an inflammatory process.

### BRIEF SUMMARY OF THE INVENTION

In accordance with the present invention, there is provided an oral composition for use in a method of preventing or reducing gingivitis or periodontitis, the method comprising administering to the oral cavity of a human or animal subject an effective amount of the oral composition consisting essentially of morin and a water-humectant phase..

Preferred features are defined in the dependent claims.

### BRIEF DESCRIPTION OF THE DRAWINGS

FIG. 1 is a graph showing anti-oxidant activity of a simple solution containing morin.

FIG. 2 is a graph showing comparative data for anti-oxidant activity of control, placebo, compositions containing morin, and a composition containing vitamin E.

### DETAILED DESCRIPTION OF THE INVENTION

The present invention arises from a finding that a composition of oral care vehicles containing morin exhibits stability and anti-oxidative efficacy and can be used in a method of preventing or reducing gingivitis or periodontitis. Also, it is found that morin exhibits an additive effect when combined with a broad spectrum antibacterial such as triclosan.

An oral composition in accordance with the present invention comprises morin as anti-oxidant. Morin (2',3,4',5,7-pentahydroxyflavone) is a phenolic compound belonging to the group of flavonoid plant dyes and has the following structure:

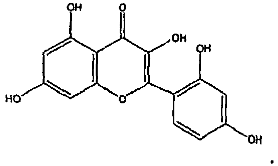

In one embodiment, an oral composition consists essentially of morin and a water-humectant phase. The oral composition containing morin is useful to alleviate tissue damage caused by oxidative free radicals. In another embodiment, an oral composition comprises morin, a water-humectant phase, and other ingredients effective to kill bacteria or to reduce inflammatory processes. Morin, can be combined with other therapeutic agents to broaden or strengthen oral hygiene efficacy of an oral composition. For example, when combined with an anti-caries agent, an oral composition containing morin and the anti-caries agent can be utilized for dual purposes, i.e., treating tooth decay and periodontal disease.

Other therapeutic agents include, but are not limited to, anticaries agents and antibacterial agents, and antibacterial enhancing agents. Though any known therapeutic agents can be used together with morin, it may be preferable to combine fluoride sources and/or triclosan with morin.

Morin is added to oral compositions in an effective amount to, thereby preventing or treating oral inflammatory diseases. Morin may be present at amount of about 0.1% to about 30%, preferably, about 0.5% to about 10% by weight of the oral composition.

An oral composition in accordance with the present invention may contain one or more antibacterial agents in addition to morin. Addition of antibacterial agents may enhance or broaden antibacterial efficacy of the dentifrice composition. Such antibacterial agents include non-cationic antibacterial agents which are based on phenolic or bisphenolic compounds, such as halogenated diphenyl ethers such as triclosan (2,4,4'-trichloro-2'-hydroxydiphenyl ether). Other useful antibacterial agents are, for example, arginate esters, or salts, cetyl pyrinidium salts, phenolic antibacterial compounds (menthol, magonol, honokiol).

Preferably, triclosan can be used together with morin to strengthen anti-oxidative efficacy and to broaden antibacterial activity of an oral formulation. An oral composition comprising morin and triclosan may not only suppress inflammatory processes by anti-oxidative activity of the composition but also kill pathogens causing dental-related diseases.

These antibacterial agents are included in the dentifrice composition at a concentration of about 0.1 % to about 30% by weight of the oral composition.

An oral composition of the present invention may also contain a source of fluoride ions or fluorine-providing ingredient, as anticaries agent in amounts sufficient to supply about 25 ppm to 5,000 ppm of fluoride ions and include inorganic fluoride salts, such as soluble alkali metal salts. In one embodiment, an oral composition comprises morin, a water-humectant phase, and a fluoride source. The formulation may be useful to prevent or treat various dental-related diseases such as, for example, tooth decay, gingivitis, and periodontitis.

In another embodiment, a fluoride source is added to an oral composition comprising morin and one or more bacterial agents to broaden the spectrum of oral care efficacy of the composition. For example, a preferred antibacterial agent may be triclosan and a preferred fluoride ion source may include sodium fluoride, potassium fluoride, sodium fluorosilicate, sodium monfluorophosphate (MFP), and ammonium fluorosilicate.

In addition to fluoride compounds, there may also be included in the oral compositions of the present inventions antitartar agents such as pyrophosphate salts including dialkali or tetraalkali metal pyrophosphate salts such as Na₄P₂O₇, K₄P₂O₇, Na₂K₂P₂O₇, Na₂H₂P₂O and K₂H₂P₂O₇, polyphosphates such as sodium tripolyphosphate, sodium hexametaphosphate and cyclic phosphates such as sodium tripolyphosphate sodium trimetaphosphate.

Synthetic anionic polycarboxylates may also be used in the oral compositions of the present invention as an efficacy enhancing agent for morin, for any antibacterial, antitartar or other active agent within the dentifrice composition. Such anionic polycarboxylates are generally employed in the form of their free acids or preferably partially or more preferably fully neutralized water soluble alkali metal (*e.g*., potassium and preferably sodium) or ammonium salts. Preferred are 1:4 to 4:1 copolymers of maleic anhydride or acid with another polymerizable ethylenically unsaturated monomer, preferably methylvinylether/maleic anhydride having a molecular weight (M.W.) of about 30,000 to about 1,800,000, and most preferably about 30,000 to about 700,000. Examples of these copolymers are available from GAF Corporation under the tradename GANTREZ®, *e.g*., AN 139 (M.W. 500,000), AN 119 (M.W. 250,000); S-97 Pharmaceutical Grade (M.W. 700,000), AN 169 (M.W. 1,200,000-1,800,000), and AN 179 (M.W. above 1,800,000).

When present, the anionic polycarboxylate is employed in amounts effective to achieve the desired enhancement of the efficacy of any antibacterial, antitartar or other active agent within the oral composition.

Orally-acceptable vehicles used to prepare dentifrice compositions of the present invention include a water-phase, containing a humectant therein. The humectant is preferably glycerin, sorbitol, xylitol, dipropylene glycol, methyl cellosolve, ethyl cellosolve, olive oil, castor oil, amyl acetate, ethyl acetate, glyceryl tristearate and benzyl benzoate and/or propylene glycol; but, other humectants and mixtures thereof may also be employed.

In the preparation of a dentifrice composition, abrasives which may be used in the practice of the present invention include silica abrasives such as precipitated silicas having a mean particle size of up to about 20 microns, such as ZEODENT® 115, marketed by J. M. Huber. Other useful dentifrice abrasives include sodium metaphosphate, potassium metaphosphate, tricalcium phosphate, dihydrated dicalcium phosphate, aluminum silicate, calcined alumina, bentonite and other siliceous materials, and combinations thereof.

Thickeners used in the dentifrice compositions of the present invention include natural and synthetic gums and colloids. Thickeners compatible with the present composition include cellulose thickeners such as carboxymethyl cellulose, hyroxyalkyl celluloses such as hydroxypropyl cellulose hydroxyethyl cellulose, gums such as xanthan gum, polyglycols of varying molecular weights sold under the tradename Polyox and polyethylene glycol. Inorganic thickeners which may be used in the practice of the present invention include amorphous silica compounds such as colloidal silicas compounds available under the trade designation CAB-O-SIL® manufactured by Cabot Corporation and distributed by Lenape Chemical, Bound Brook, N.J.; ZEODENT® 165 from J. M. Huber Chemicals Division, Havre de Grace, Md. 21078; and SYLODENT® 15, available from Davison Chemical Division of W. R. Grace Corporation, Baltimore, Md. 21203. Other inorganic thickeners include natural and synthetic clays, lithium magnesium silicate and magnesium aluminum silicate.

Surfactants are used in the oral compositions of the present invention to achieve increased prophylactic action and render the compositions more cosmetically acceptable. The surfactant is preferably a detersive material which imparts to the composition detersive and foaming properties.

The oral composition of the present invention may also contain flavoring agents and/or breath freshening antiplaque actives. Flavoring agents which are used in the practice of the present invention include essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint and spearmint.

The sweetener content will normally be that of an artificial or synthetic sweetener (non-sugar).

Various other materials may be incorporated in the oral compositions of this invention, including desensitizers, such as potassium nitrate; whitening agents, such as hydrogen peroxide, calcium peroxide and urea peroxide; preservatives; silicones; and chlorophyll compounds. These additives, when present, are incorporated in the oral compositions of the present invention in amounts which do not substantially adversely affect the properties and characteristics desired.

In the present invention, an oral composition containing morin is for use in a method to prevent or treat dental-related diseases, particularly gingivitis and/or periodontitis, by administering to the oral cavity of human or animal the composition. The method using a morin composition is especially useful to prevent or treat dental inflammatory diseases such as gingivitis and periodontitis since the present dentifrice compositions have superior anti-oxidative efficacy. To broaden the scope of target disease to be treated, one or more other therapeutic agents can be added to the morin composition. For example, a composition comprising morin and an anti-caries agent can be used in a method to prevent or treat tooth decay, gingivitis, and periodontitis. Preferably, the dentifrice composition to be administered may contain one or more conventional antibacterial agents such as triclosan, fluoride, an arginate ester, solbrol and cetyl pyrinidium salts. An oral composition containing morin to be used for the method may be prepared by suitably mixing other ingredients as mentioned above.

For effective treatment of dental-related disease, morin may be administered to the oral cavity of human or animal in amount of about 10 ppm to about 10,000 ppm, preferably, about 100 ppm to about 5,000 ppm. And a therapeutic agent used together with morin may be administered to human or animal in amount of about 10 ppm to about 10,000 ppm, preferably, about 100 ppm to about 5,000 ppm.

The oral composition to be used in the method can be further processed to different types of final products so as to meet consumer needs. For example, the composition to be administered to human or animal may be in a form selected from pet treats, toys, breath strips, mouthwash, toothpaste, liquid whitener, chewing gum, bead, chew, and lozenge.

The invention is further illustrated but not limited by the following Examples. Variations of the following examples are possible without departing from the scope of the invention.

### EXAMPLES

### Example 1

The anti-oxidant efficacy of morin in simple solutions was tested using the LPO-CC Kamiya Bioscience kit which is a spectroscopy-based assay that measures the amount of methylene blue produced. Reaction processes in the kit used can be summarized as follows. Cumene hydroperoxide (CHO) is combined with an enzyme mixture of ascorbic oxidase and lipoprotein lipase in order to produce lipid peroxide. Samples and standards are then combined with a second reagent containing methyl carbamate (MCDP) and hemoglobin. In the presence of hemoglobin, lipid peroxides are converted to lipid alcohols which in turn convert the MCDP to methylene blue that can be read at 674nm. This translates to decreased color intensity which is measured by spectroscopy at 674nm. If the active is an anti-oxidant, it should drop the optical density reading from that which was taken from the standard curve. In other words, the lower the optical density reading the better the anti-oxidant efficacy.

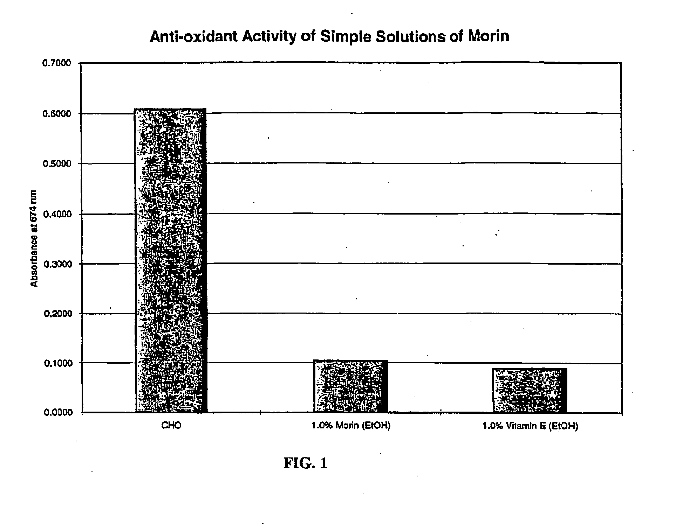

A simple composition containing 1.0% by weight of morin was tested by the kit above and compared with a simple composition containing 1.0% by weight of vitamin E. Fig. 1 illustrates the result of the experiment. As shown in Fig. 1, morin exhibited anti-oxidative efficacy as good as positive control vitamin E.

### Example 2

Anti-oxidant efficacy of oral compositions containing morin was tested, using the LPO-CC Kamiya Bioscience kit. A dentifrice base was prepared using the ingredients listed in Table 1 below:

**TABLE I**

| **Ingredients** | **Weight (g)** |
|---|---|
| Water | 16.3 |
| Sodium saccharin | 0.3 |
| Sodium fluoride | 0.2 |
| Glycerin (99.5%) | 20.0 |
| Sodium carboxymethyl cellulose | 1.1 |
| Iota carrageenan | 0.4 |
| Titanium dioxide | 0.5 |
| Sorbitol non-browning (70%) | 20.9 |
| GANTREZ® S-97 (15%) | 15.0 |
| Sodium hydroxide (50%) | 1.2 |
| ZEODENT® 115 | 20.0 |
| ZEODENT® 165 | 1.5 |
| Active | 0.1 |
| Flavor | 1.0 |
| Sodium lauryl sulfate powder | 1.5 |
| **TOTAL** | **100.0** |

Three types of oral compositions, Compositions A, B, and C, were formulated based upon the common dentifrice base prepared above. Composition A was formulated to contain 0.3% morin, 1.0% flavor, 1.5% sodium lauryl sulfate powder, 1.5% Zeodent 165, 20.0% Zeodent 115, and 75.4% dentifrice base. Composition B was formulated to contain 0.3% triclosan, in addition to the ingredients of composition A. Composition C is similar to composition A except that it employed 0.3% vitamin E as anti-oxidative agent instead of morin. The components of the compositions used in a comparative experiment are summarized in the chart below:

**TABLE II**

| | **Weight (%)** | | | |
|---|---|---|---|---|
| **Composition No.** | **Placebo** | **A** | **B** | C |
| **Ingredients** | | | | |
| Triclosan | - | - | 0.3 | - |
| Morin | - | 0.3 | 0.3 | - |
| Vitamin E | - | - | - | 0.3 |
| Flavor | 1.0 | 1.0 | 1.0 | 1.0 |
| Sodium lauryl sulfate powder | 1.5 | 1.5 | 1.5 | 1.5 |
| ZEODENT® 165 | 1.5 | 1.5 | 1.5 | 1.5 |
| ZEODENT® 115 | 20 | 20.0 | 20.0 | 20.0 |
| Dentifrice base | 76 | 75.7 | 75.4 | 75.7 |
| **TOTAL** | **100.0** | **100.0** | **100.0** | **100.0** |

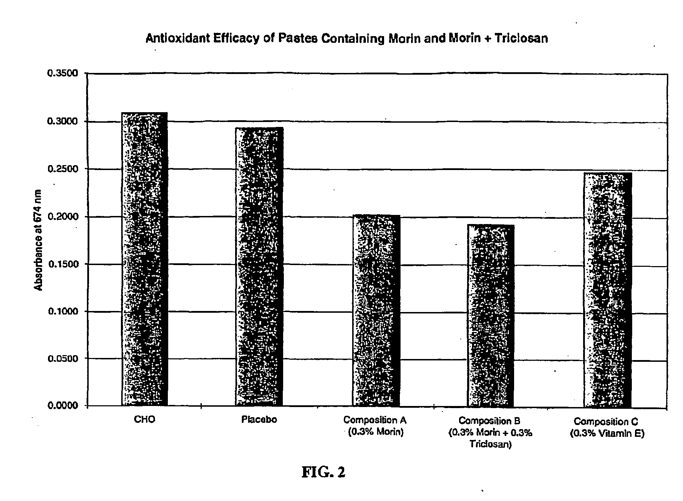

Anti-oxidative efficacy of each composition was evaluated with the same protocol as used in Example 1. Fig. 2 shows the anti-oxidative efficacy of the compositions. Composition A containing morin, only, as anti-oxidant, exhibited anti-oxidant efficacy well over the control paste (placebo). Furthermore, composition A was shown to be superior to composition C containing vitamin E in terms of anti-oxidative efficacy. In addition, composition B comprising morin and triclosan was found to have slightly stronger anti-oxidative efficacy than composition A.

Although the invention has been described with reference to specific examples, it will be apparent to one skilled in the art that various modifications may be made thereto which fall within its scope.

## Claims

1. An oral composition for use in a method of preventing or reducing gingivitis or periodontitis, the method comprising administering to the oral cavity of a human or animal subject an effective amount of the oral composition consisting essentially of morin and a water-humectant phase.

2. The oral composition according to claim 1, wherein the morin is administered to the oral cavity in amount of 10 ppm to 10,000 ppm.

3. The oral composition according to claim 1, wherein the oral composition further comprises an antibacterial agent, an antibacterial enhancing agent, and a fluoride ion source.

4. The oral composition according to claim 3, wherein the morin is administered to the oral cavity in amount of 100 ppm to 5,000 ppm.

5. The oral composition of any foregoing claim, wherein the composition is provided in a form selected from a mouthwash, oral strip, toothpaste, liquid whitener, chewing gum, bead, chew, lozenge, pet treats and toys, and spray.

6. The oral composition of claim 1, further comprising a therapeutic agent selected from herbs, moisturizers, whitening, anti-attachment agents, triclosan, an arginate ester, solbrol and cetyl pyrinidium salts.

7. The oral composition of claim 1, wherein the oral composition further comprises a fluoride ion source.

8. The oral composition of claim 7, wherein the fluoride ion source is selected from sodium fluoride, potassium fluoride, ammonium fluoride, calcium fluoride, cuprous fluoride, zinc fluoride, stannous fluoride, and barium fluoride.

9. The oral composition of claim 1, wherein the humectant of the water-humectant phase is selected from propylene glycol, dipropylene glycol, methyl cellosolve, ethyl cellosolve, olive oil, castor oil, amyl acetate, ethyl acetate, glyceryl tristearate and benzyl benzoate.

10. The oral composition of claim 1, wherein the oral composition further comprises an ingredient selected from a polishing agent, a surfactant, a flavoring agent, and a sweetener.

## Patentansprüche

1. Mundpflegezusammensetzung zur Verwendung in einem Verfahren zur Vorbeugung oder Verminderung von Zahnfleischentzündung oder Wurzelhautentzündung, wobei das Verfahren die Verabreichung einer effektiven Menge der Mundpflegezusammensetzung, die im Wesentlichen aus Morin und einer Wasser-Feuchthaltemittelphase besteht, in die Mundhöhle eines Menschen oder Tieres umfaßt.

2. Mundpflegezusammensetzung nach Anspruch 1, wobei Morin in einer Menge von 10 ppm bis 10 000 ppm in die Mundhöhle verabreicht wird.

3. Mundpflegezusammensetzung nach Anspruch 1, die weiterhin ein antibakterielles Mittel, ein antibakteriell Mittel steigerndes Mittel und eine Fluorionenquelle umfaßt.

4. Mundpflegezusammensetzung nach Anspruch 3, wobei Morin in einer Menge von 100 ppm bis 5 000 ppm in die Mundhöhle verabreicht wird.

5. Mundpflegezusammensetzung nach einem der vorgehenden Ansprüche, die in einer Form bereitgestellt wird, die aus einer Mundspülung, einem Mundpflegestreifen, Zahnpasta, einem flüssigen Bleichmittel, Kaugummi, Kügelchen, Priem, Pastillen, Tiernahrung und -spielzeugen, und Spray ausgewählt ist.

6. Mundpflegezusammensetzung nach Anspruch 1, die weiterhin ein therapeutisches Mittel umfaßt, das aus Kräutern, Feuchtigkeitsmitteln, Bleichmitteln, Antihaftmitteln, Triclosan, einem Arginatester, Solbrol und Cetylpyridiniumsalzen ausgewählt ist.

7. Mundpflegezusammensetzung nach Anspruch 1, die weiterhin eine Fluoridionenquelle umfaßt.

8. Mundpflegezusammensetzung nach Anspruch 7, wobei die Fluoridionenquelle aus Natriumfluorid, Kaliumfluorid, Ammoniumfluorid, Calcuifluorid, Kupferfluorid, Zinkfluorid, Zinnfluorid und Bariumfluorid ausgewählt ist.

9. Mundpflegezusammensetzung nach Anspruch 1, wobei das Feuchthaltemittel der Wasser-Feuchthaltemittelphase aus Propylenglykol, Dipropylenglykol, Methylcellusolve, Ethylcellusolve, Olivenöl, Castoröl, Amylacetat, Ethylacetat, Glyceryltristearat und Benzylbenzoat ausgewählt ist.

10. Mundpflegezusammensetzung nach Anspruch 1, die weiterhin einen Inhaltsstoff umfaßt, der aus einem Poliermittel, einem Tensid, einem Geschmacksmittel und einem Süßungsmittel ausgewählt ist.

## Revendications

1. Composition orale pour une utilisation dans une méthode de prévention ou de réduction d'une gingivite ou d'une parodontite, la méthode comprenant l'administration dans la cavité buccale d'un sujet humain ou animal d'une quantité efficace de la composition orale essentiellement constituée de morine et d'une phase aqueuse contenant un humectant.

2. Composition orale selon la revendication 1, dans laquelle la morine est administrée dans la cavité buccale en une quantité de 10 ppm à 10 000 ppm.

3. Composition orale selon la revendication 1, la composition orale comprenant en outre un agent antibactérien, un agent améliorant l'effet antibactérien, et une source d'ion fluorure.

4. Composition orale selon la revendication 3, dans laquelle la morine est administrée dans la cavité buccale en une quantité de 100 ppm à 5000 ppm.

5. Composition orale selon l'une quelconque des revendications précédentes, la composition étant sous une forme choisie parmi un bain de bouche, une bande buccale, un dentifrice, un blanchisseur liquide, une gomme à mâcher, des billes, un composé à mâcher, une pastille, des friandises et des jouets pour animaux domestiques, et un vaporisateur.

6. Composition orale selon la revendication 1, comprenant en outre un agent thérapeutique choisi parmi les herbes, les agents hydratants, les agents blanchissants, les agents anti-attachement, le triclosan, un ester d'arginate, le solbrol et les sels de cétylpyrinidium.

7. Composition orale selon la revendication 1, la composition orale comprenant en outre une source d'ion fluorure.

8. Composition orale selon la revendication 7, dans laquelle la source d'ion fluorure est choisie parmi le fluorure de sodium, le fluorure de potassium, le fluorure d'ammonium, le fluorure de calcium, le fluorure de cuivre, le fluorure de zinc, le fluorure d'étain et le fluorure de baryum.

9. Composition orale selon la revendication 1, dans laquelle l'humectant de la phase aqueuse contenant un humectant est choisi parmi le propylène glycol, le dipropylène glycol, le méthylcellosolve, l'éthylcellosolve, l'huile déclive, l'huile de ricin, l'acétate d'amyle, l'acétate d'éthyle, le tristéarate de glycéryle et le benzoate de benzyle.

10. Composition orale selon la revendication 1, la composition orale comprenant en outre un ingrédient choisi parmi un agent de polissage, un tensioactif, un agent aromatisant et un édulcorant.
